(19) **Europäisches Patentamt / European Patent Office / Office européen des brevets**

(11) **EP 3 709 878 B1**

(12) **FASCICULE DE BREVET EUROPEEN**

(45) Date de publication et mention de la délivrance du brevet:
**19.03.2025 Bulletin 2025/12**

(21) Numéro de dépôt: **18825744.8**

(22) Date de dépôt: **15.11.2018**

(51) Classification Internationale des Brevets (IPC):
**A61B 5/296** (2021.01) **A61B 5/389** (2021.01)

(52) Classification Coopérative des Brevets (CPC):
**A61B 5/6831; A61B 5/316; A61B 5/389; A61B 5/6823;** A61B 5/7235

(86) Numéro de dépôt international:
**PCT/FR2018/052846**

(87) Numéro de publication internationale:
**WO 2019/097166 (23.05.2019 Gazette 2019/21)**

(54) **DISPOSITIF ET PROCÉDÉ DE DÉTECTION D'ACTIVITÉ MUSCULAIRE D'UN OU PLUSIEURS MUSCLES PROFONDS PAR MESURES NON INVASIVES**

VORRICHTUNG UND VERFAHREN ZUR ERFASSUNG DER MUSKELAKTIVITÄT EINER ODER MEHRERER TIEFENMUSKELN DURCH NICHTINVASIVE MESSUNGEN

DEVICE AND METHOD FOR DETECTING MUSCULAR ACTIVITY OF ONE OR MORE DEEP MUSCLES BY NON-INVASIVE MEASUREMENTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.11.2017 FR 1771225**

(43) Date de publication de la demande:
**23.09.2020 Bulletin 2020/39**

(73) Titulaires:
• **Pautard, Caroline**
  **35760 Saint-Gregoire (FR)**
• **Blueback**
  **35510 Cession-Sévigné (FR)**

(72) Inventeurs:
• **Pautard, Caroline**
  **35760 Saint-Gregoire (FR)**
• **Jouanneau, Clément**
  **35760 Saint-Gregoire (FR)**

(74) Mandataire: **Novagraaf Technologies**
  **Bâtiment O2**
  **2, rue Sarah Bernhardt**
  **CS90017**
  **92665 Asnières-sur-Seine Cedex (FR)**

(56) Documents cités:
**US-A1- 2012 184 838   US-B2- 9 687 168**

• **CHANTHAPETCH P ET AL: "Abdominal muscle activity during abdominal hollowing in four starting positions", MANUAL THERAPY, CHURCHILL LIVINGSTONE, AMSTERDAM, NL, vol. 14, no. 6, 1 December 2009 (2009-12-01), pages 642 - 646, XP026740087, ISSN: 1356-689X, [retrieved on 20090228], DOI: 10.1016/ J.MATH.2008.12.009**
• **SANCHEZ-ZURIAGA D ET AL: "Trunk Muscle Activation Patterns and Spine Kinematics When Using an Oscillating Blade: Influence of Different Postures and Blade Orientations", ARCHIVES OF PHYSICAL MEDICINE AND REHABILITATION, W.B. SAUNDERS, UNITED STATES, vol. 90, no. 6, 1 June 2009 (2009-06-01), pages 1055 - 1060, XP026162337, ISSN: 0003-9993, [retrieved on 20090527], DOI: 10.1016/J.APMR.2008.12.015**
• **STUART MCGILL ET AL: "Appropriately placed surface EMG electrodes reflect deep muscle activity", JOURNAL OF BIOMECHANICS, vol. 29, no. 11, 1 November 1996 (1996-11-01), pages 1503 - 1507, XP055508163, DOI: 10.1016/ 0021-9290(96)84547-7**

• EVA SWINNEN ET AL: "Methodology of electromyographic analysis of the trunk muscles during walking in healthy subjects: A literature review", JOURNAL OF ELECTROMYOGRAPHY AND KINESIOLOGY, ELSEVIER, AMSTERDAM, NL, vol. 22, no. 1, 13 April 2011 (2011-04-13), pages 1 - 12, XP028346678, ISSN: 1050-6411, [retrieved on 20110427], DOI: 10.1016/ J.JELEKIN.2011.04.005

**Description**

**Domaine technique**

La présente demande concerne un dispositif et un procédé permettant directement et instantanément de mesurer et de quantifier le niveau d'activité musculaire de un ou plusieurs muscles profonds de la sangle abdominale d'un sujet par des mesures électromyographiques non invasives.

Le domaine de demande concerne une variété d'applications comme la rééducation fonctionnelle, le sport, le bien-être, l'esthétique ou encore le diagnostic fonctionnel.

**Etat de la technique**

Dans le domaine de la mesure non invasive de l'activité musculaire, on connait la mesure par électromyographie (EMG), consistant à positionner des électrodes adhésives sur la peau du patient, stratégiquement par rapport à l'anatomie du muscle pour lequel on cherche à enregistrer l'activité musculaire.

Cette technique est limitée car le signal recueilli ne peut être spécifique du muscle ciblé que si ledit muscle est positionné juste en dessous de l'électrode de mesure. Elle s'applique donc principalement pour les muscles dits superficiels, situés juste en dessous de la peau. Le projet SENIAM (http://www.seniam.org/) a d'ailleurs été conduit dans le but de consolider la recherche appliquée dans le domaine de l'électromyographie de surface, ce qui a résulté en la publication de recommandations européennes concernant les capteurs, leur positionnement optimal par muscle ciblé et les méthodes de traitement de signal adaptées. Ces recommandations concernent donc 30 muscles individuels, mais ne comprennent pas les muscles profonds visés par la présente invention. Parallèlement, il y a eu plusieurs tentatives pour mesurer l'activité musculaire de muscles profonds à partir d'électrodes EMG de surface.

McGill et al (1996, J. Biomechanics, Vol 29, No 11, pp 1503-1507) ont étudié la possibilité de mesurer l'activité musculaire de 5 muscles spécifiques (le psoas, l'oblique externe, l'oblique interne, le transverse abdominal et le carré de lombes) à partir d'une unique électrode EMG de surface positionnée de façon stratégique pour chaque muscle. Cette étude montre que certains sites anatomiques de surface pourraient être utilisés pour refléter l'activité musculaire de muscles profonds comme le carré des lombes et le psoas. Cependant, l'erreur sur la mesure est de 6% à 12%. De plus, les conclusions montrent que la localisation qui permettrait de mesurer (avec une erreur de 15%) le transverse abdominal est la même que celle qui représente au mieux l'activité musculaire de l'oblique interne (15cm en latéral du nombril, juste au-dessus du ligament inguinal). Ainsi, cette localisation n'est pas spécifique d'un de ces deux muscles et la méthode ainsi décrite ne permet pas de répondre au problème technique résolu par la présente invention.

Jesinger et Stonick (1994, 6th IEEE DSP Workshop Proc., p57-60) proposent de résoudre un problème inverse basé sur une modélisation en éléments finis de l'activité électrique des muscles profonds et de sa propagation jusqu'à la surface de la peau. Cette méthode, comme d'autres méthodes basées sur des principes similaires de résolution inverse issues d'un modèle, nécessite un nombre élevé d'électrodes (plus de 100) pour obtenir la quantité d'information suffisante permettant de résoudre le problème mathématique. Ces méthodes sont d'ailleurs principalement utilisées à des fins de recherche et ne sont pas nécessairement adaptées à des applications industrielles pour lesquelles les calculs doivent être faits en temps réel avec un dispositif simple d'utilisation. Dans la présente invention, a contrario, le dispositif comprend au maximum 5 électrodes de mesures et permet d'afficher en temps réel l'activité de chaque muscle profond de la sangle abdominale, comme le transverse abdominal ou l'oblique interne.

L'invention décrite dans le brevet US 9,687,168 consiste en une méthode d'électromyographie de surface des muscles profonds. L'invention consiste à disposer une matrice d'électrodes mono-polaires encerclant la circonférence d'une partie du corps dans laquelle est incluse le muscle profond que l'on cherche à investiguer. L'exemple d'application retenu concerne la mesure du muscle brachial du bras, en disposant de deux matrices de 6 électrodes chacune, configurées en deux cercles concentriques autour du bras. Ainsi, si l'on cherche à appliquer la méthode au cas des muscles profonds de la sangle abdominale, le nombre d'électrodes de la matrice est nécessairement supérieur à 5, même pour une personne de faible corpulence. De plus, ce brevet n'enseigne pas le mode de réalisation permettant d'appliquer la méthode pour les muscles profonds de la sangle abdominale, étant donné qu'une phase de calibration spécifique de la distribution des muscles de la zone investiguée est nécessaire à l'application de l'analyse en composants indépendants réalisée par la suite.

A l'heure actuelle, il n'existe donc aucun dispositif et aucune méthode permettant de mesurer l'activité musculaire spécifique à chaque muscle profond de la sangle abdominale, basés sur des mesures électromyographiques non invasives, par un arrangement non circulaire de 1 à 5 capteurs EMG de surface placés sur la peau du sujet en mouvement.

Un but de la présente demande est de fournir un dispositif et une méthode permettant à un utilisateur réalisant des mouvements, de visualiser et d'évaluer en temps réel l'activité musculaire des différents muscles profonds de la sangle abdominale (Transverse abdominal et Oblique Interne).

Un autre but de la présente demande est de fournir un dispositif permettant de visualiser et d'évaluer en temps réel l'activité musculaire des différents muscles profonds de la sangle abdominale qui soit facilement manipulable par l'utilisateur.

**Description de l'invention**

A cet effet, l'invention a pour objet une méthode selon le revendication 1.

Par exemple, dans le cas d'une mise en œuvre de l'invention avec 2 capteurs, ceux-ci peuvent être placés sur la peau d'un sujet selon les indications suivantes :

- Un capteur positionné dans une bande située sous la ligne formée par les deux épines iliaques antéro-supérieures, et au-dessus de la symphyse pubienne. Ce capteur peut être dans ce cas positionné entre les deux ligaments inguinaux.

Par exemple, dans le cas d'une mise en œuvre de l'invention avec 3 capteurs, ceux-ci peuvent être placés sur la peau d'un sujet selon les indications suivantes :

- Deux capteurs positionnés de façon symétrique par rapport à l'axe ombilic/pubis du sujet, dans une bande située sous la ligne formée par les deux épines iliaques antéro-supérieures, et au-dessus de la symphyse pubienne. Ces deux capteurs peuvent dans ce cas être positionnés être entre les deux ligaments inguinaux, et
- Un capteur positionné sur le muscle Grand Droit.

Par exemple, dans le cas d'une mise en œuvre de l'invention avec 4 capteurs, ceux-ci peuvent être placés sur la peau d'un sujet selon les indications suivantes :

- Deux capteurs positionnés de façon symétrique par rapport à l'axe ombilic/pubis du sujet, dans une bande située sous la ligne formée par les deux épines iliaques antéro-supérieures, et au-dessus de la symphyse pubienne. Ces deux capteurs peuvent dans ce cas être positionnés être entre les deux ligaments inguinaux, et
- Deux capteurs positionnés sur le muscle Grand Droit.

Par exemple, dans le cas d'une mise en œuvre de l'invention avec 5 capteurs, ceux-ci peuvent être placés sur la peau d'un sujet selon les indications suivantes :

- Deux capteurs positionnés de façon symétrique par rapport à l'axe ombilic/pubis du sujet, dans une bande située sous la ligne formée par les deux épines iliaques antéro-supérieures, et au-dessus de la symphyse pubienne. Ces deux capteurs peuvent dans ce cas être positionnés être entre les deux ligaments inguinaux,
- Deux capteurs positionnés sur le muscle Grand Droit, et
- Un capteur en face dorsale, sur une ligne reliant l'épine iliaque antéro-supérieure et l'espace intervertébral L1-L2, au niveau de l'apophyse épineuse L5.

Les capteurs peuvent être avantageusement placés de manière à mesurer spécifiquement l'activité musculaire de chaque muscle profond ciblé. Selon l'invention, les capteurs sont positionnés en un arrangement non circulaire le long du muscle ciblé.

Avantageusement, mais facultativement, l'invention comprend au moins l'une des caractéristiques suivantes :

- Des moyens de visualisation des signaux acquis, enregistrés, traités, ou des indices calculés. Il peut s'agir par exemple de tout moyen adapté connu de l'homme du métier, comme par exemple un moniteur d'ordinateur, une tablette tactile ou un écran de téléphone portable.

- Des moyens d'enregistrements des signaux à une fréquence d'échantillonnage comprise en 500Hz et 3000Hz. Il peut s'agir par exemple de tout moyen adapté connu de l'homme du métier, comme par exemple une chaine d'acquisition de conversion analogique/digital haute résolution combinée à une mémoire.
- Des moyens d'appliquer des algorithmes de traitement de signal sélectionnés parmi des algorithmes de lissage, de seuillage, de moyennage, de normalisation, d'analyse fréquentielle, d'analyse de corrélation temporelle entre au moins deux signaux, ou sur une analyse en composantes indépendantes, ou une combinaison de ces méthodes.
- Des moyens de communication sans fil pour transmettre les signaux acquis vers la mémoire. Il peut s'agir par exemple de tout moyen adapté connu de l'homme du métier, comme par exemple le protocole Bluetooth, le protocole Bluetooth Low Energy ou le protocole WIFI ou LoRa.
- Des moyens de mesure de l'activité musculaire d'un ou plusieurs muscles profonds de la sangle abdominale d'un sujet à partir d'électrodes EMG bipolaires. Il peut s'agir de tout moyen adapté connu de l'homme du métier, comme par exemple une EMG bipolaire, par exemple les électrodes 3M positionnées sur le muscle.

L'invention concerne une méthode de mesure de l'activité musculaire de l'oblique interne et du transverse abdominal d'un sujet à partir de capteurs électromyographiques surfaciques non invasifs.

La présente demande concerne également un dispositif de mesure de l'activité musculaire spécifique d'un ou plusieurs muscles profonds de la sangle abdominale d'un sujet à partir de capteurs électromyographiques surfaciques non invasifs comprenant :

- Des moyens d'acquisition des signaux électriques issus d'au moins deux capteurs, le dispositif étant caractérisé par le fait que le nombre de capteurs est compris entre 2 et 5.
- Des moyens d'enregistrement des signaux électriques acquis grâce aux moyens d'acquisition dans une mémoire.
- Des moyens de traitement des signaux enregistrés par les moyens d'enregistrement, permettant d'appliquer sur les signaux enregistrés une combinaison d'algorithmes de traitement de signal pour délivrer ainsi une pluralité de signaux traités.
- Des moyens de calcul d'au moins un indice représentatif de l'activité musculaire de chacun des au moins un muscle profond de la sangle abdominale du sujet et son évolution temporelle.
- Des moyens de visualisation des signaux acquis, enregistrés, traités, ou des indices calculés.

Au sens de la présente demande, les moyens d'acquisition des signaux électriques, les moyens d'enregistrement des signaux électriques, les moyens de traitement des signaux enregistrés, et les moyens de visualisation des signaux peuvent être tout moyen adapté connu de l'homme du métier, comme par exemple ceux cités précédemment.

Au sens de la présente demande, les moyens de calcul peuvent être tout moyen adapté connu de l'homme du métier, comme par exemple l'exécution des algorithmes au sein d'environnements de calcul numérique ou au sein d'un processeur rapide convenablement programmé.

Avantageusement, la méthode et le dispositif permettent de mesurer l'activité musculaire de plusieurs muscles profonds (notés N) simultanément, tout en obtenant un résultat spécifique, c'est-à-dire individualisé, pour chaque muscle. En d'autres termes, la méthode et le dispositif permettent de réaliser N mesures spécifiques à chacun des N muscles visés, et d'obtenir la valeur de N activités musculaires. Ainsi, il est possible de mesurer de manière spécifique, c'est-à-dire individualisée, l'activité soit d'un seul muscle profond, par exemple le muscle transverse, ou bien de plusieurs muscles profonds en même temps, par exemple le muscle transverse et l'oblique interne, tout en pouvant attribuer spécifiquement à chacun des muscles la valeur de l'activité musculaire mesurée pour ce muscle. Ce résultat est tout à fait différent et avantageux par rapport aux méthodes et dispositifs de l'état de la technique, qui permettent simplement de mesurer l'activité musculaire d'un groupe de muscles profonds, sans savoir quel est ou quels sont les muscles impliqués dans ce résultat, ni en quelle proportion.

La présente demande concerne aussi les programmes d'ordinateur stockés sur un support d'informations, lesdits programmes comportant des instructions permettant de mettre en œuvre les méthodes précédemment décrites, lorsqu'ils sont chargés et exécutés par un système informatique.

Définitions

Dans la présente demande, les termes ci-dessous sont définis de la manière suivante :
On entend par « activité musculaire spécifique d'un ou plusieurs muscles », au sens de la présente invention, l'activité musculaire individuelle de chacun des muscles dont la mesure de l'activité musculaire est réalisée, que la mesure soit réalisée sur un seul muscle, ou sur plusieurs muscles en même temps. L'activité musculaire spécifique est donc une valeur individualisée de l'activité musculaire propre à chacun des muscles soumis à la mesure.

On entend par « capteur », au sens de la présente invention, tout dispositif permettant de mesurer l'activité musculaire de muscles profonds. Il peut s'agir par exemple de de tout capteur de surface comme des électrodes d'électromyographie (EMG) de surface, de type unipolaire et/ou bipolaire, ou encore d'une sonde échographique, ou bien d'EMG par aiguille. Par exemple, le nombre de capteurs utilisés dans l'étape d'acquisition des signaux électriques peut être de 2, ou de 3, ou de 4, ou de 5.

« Muscles profonds » concerne les tissus biologiques composés de myocytes de type squelettiques localisés sous au moins un autre tissu biologique composé de myocytes.

« Sangle Abdominale » concerne la zone délimitée par les dernières cotes d'un sujet humain et la symphyse pubienne. Ainsi, dans le sens de l'invention, les muscles de la sangle abdominale concernent donc les muscles squelettiques contenus dans cette zone, préférentiellement mais ne se limitant pas, aux muscles: Transverse Abdominal, Multifidus, Oblique Interne, Grands Droits et Oblique Externe.

« Draw-in » concerne un exercice de recrutement du muscle transverse abdominal consistant à demander au sujet allongé, genoux à 90° posés sur une chaise (ou fléchis pieds au sol), d'effectuer une expiration forcée maximale lente et contrôlée en rentrant le ventre, puis de tenir cette contraction pendant 5 secondes tout en inspirant uniquement en thoracique.

« Crunch bloqué » concerne un exercice de recrutement des muscles de la sangle abdominale consistant à demander au sujet allongé sur le dos, genoux à 90° sur un support (ou fléchis), bras derrière la tête, de tenter de relever le buste pendant qu'un opérateur bloque le mouvement (via une pression sur les épaules) laissant ainsi le sujet relever son buste d'environ 30° par rapport au sol.

Présentation des dessins

D'autres caractéristiques, buts et avantages de la présente demande apparaîtront à la lecture de la description suivante d'un exemple non limitatif de mise en œuvre, donné au regard des figures :

La figure 1 est un diagramme représentatif d'un dispositif particulier et non limitatif de mise en œuvre de la méthode de mesure de l'activité musculaire d'un ou plusieurs muscles profonds de la sangle abdominale d'un sujet à partir de capteurs électromyographiques surfaciques non invasifs

La figure 2 est une représentation schématique d'un agencement particulier mais non limitatif de 5 électrodes de mesure permettant la mise en œuvre de l'invention

La figure 3 est une représentation schématique de l'anatomie musculaire de la sangle abdominale

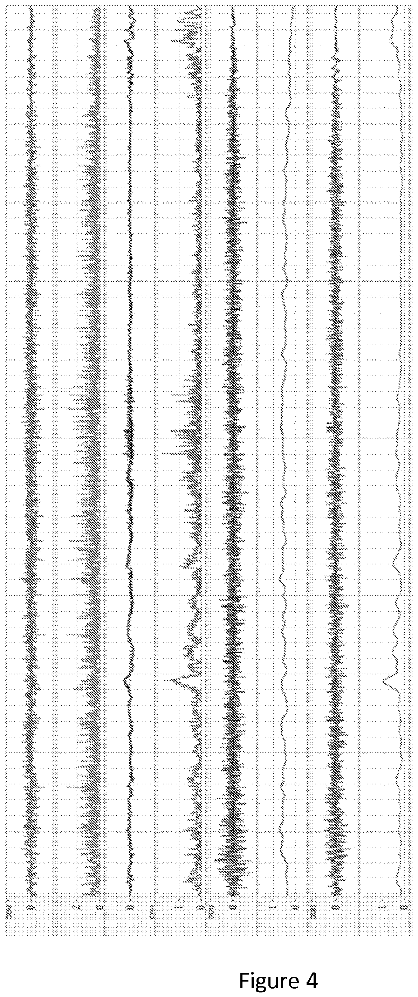

La figure 4 est une représentation d'enregistrements EMG effectués lors de tests préliminaires, utilisant l'agencement d'électrodes décrit selon la figure 2

La figure 5 est une représentation de l'évolution temporelle d'un indice d'activité musculaire pour le muscle transverse abdominal obtenu par l'application de la méthode sur deux individus distincts pendant l'exécution de manœuvres de « Draw-In » répétées

La figure 6 est une représentation schématique d'un agencement particulier mais non limitatif de 5 électrodes de mesure permettant la mise en œuvre de l'invention.

La figure 7 est une représentation schématique d'un agencement particulier mais non limitatif de 3 électrodes de mesure permettant la mise en œuvre de l'invention.

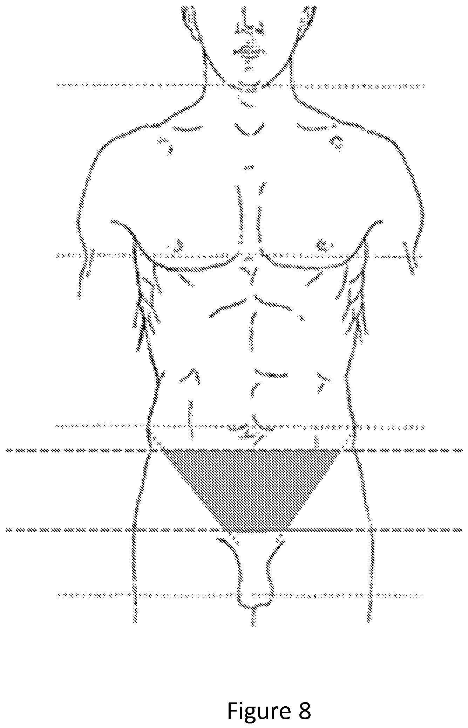

La figure 8 est une représentation schématique d'un agencement généralisé des électrodes de mesure dans une bande située sous la ligne formée par les deux épines iliaques antéro-supérieures et au-dessus de la symphyse pubienne permettant la mise en œuvre de l'invention.

**Description détaillée Exemple de réalisation**

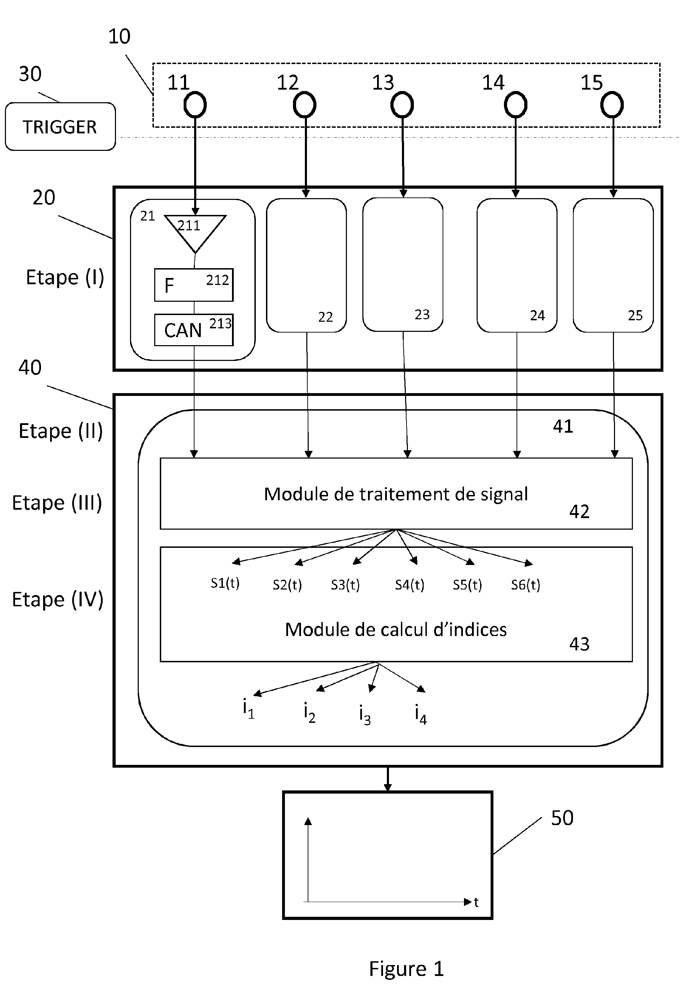

En référence à la figure 1, le dispositif permet la mise en œuvre d'une méthode de mesure de l'activité musculaire d'un ou plusieurs muscles profonds de la sangle abdominale d'un sujet à partir de capteurs électromyographiques surfaciques **10** non invasifs comprenant :

- Une étape **I** d'acquisition des signaux électriques issus d'au moins deux capteurs **10,** la méthode étant caractérisée par le fait que le nombre de capteurs est compris entre 2 et 5,
- Une étape **II** d'enregistrement des signaux électriques acquis pendant l'étape **I** dans une mémoire **41,**
- Une étape **III** de traitement des signaux enregistrés à l'étape **II** basée sur l'application sur les signaux enregistrés à l'étape **II** d'une combinaison d'algorithmes de traitement de signal dans un module de traitement de signal **42** et délivrant ainsi une pluralité de signaux traités ($s_1(t)$, $s_2(t)$, $s_3t)...s_n(t)$),
- Une étape **IV** de calcul d'au moins un indice représentatif de l'activité musculaire d'au moins un muscle profond de la sangle abdominale du sujet et son évolution temporelle dans un module de calcul d'indice **43.**

Dans l'exemple décrit, représentatif mais non limitatif, le dispositif comporte 5 électrodes de mesure EMG de surface **11, 12, 13, 14 et 15.** Dans l'exemple décrit il s'agit d'électrodes bipolaires (système *wireless Miniwave Infinity*). L'acquisition simultanée des 5 capteurs est synchronisée grâce au déclenchement d'un trigger **30.** Chaque électrode de mesure est reliée à une unité de prétraitement **20.** Cette unité **20** comprend ensuite une chaîne d'acquisition par électrode **21, 22, 23, 24 et 25.** Chaque chaîne de traitement contenant un module d'amplification, un module de filtrage et un module de conversion analogique-digitale. La figure représente de manière détaillée uniquement la chaine d'acquisition **21** de l'électrode **11,** comprenant donc le module d'amplification **211,** le module de filtrage **212** ainsi qu'un module de conversion analogique-numérique **213.** Dans l'exemple décrit, les filtres appliqués aux signaux sont ceux classiquement utilisés pour l'acquisition des signaux EMG et connus de l'homme du métier. La conversion analogique-digitale a été effectuée à 2kHz sur 16bits.

L'unité de prétraitement **20** contient des moyens de communication pour le transfert des données ainsi acquises pendant l'étape I à un processeur **40.**

Avantageusement, mais facultativement, ces moyens de communication peuvent mettre en œuvre des protocoles de communication sans fil.

Le processeur **40** contient une mémoire **41** dans laquelle les signaux acquis seront stockés pendant l'étape II d'enregistrement des données.

Cette mémoire appelle également différents algorithmes de traitement de signal utilisés par le module de traitement des signaux **42** pour mener à bien l'étape III de traitement des signaux. L'application de ces algorithmes de traitement de signal va conduire au calcul de signaux traités $s_i(t)$, i étant compris entre 1 et 10, puis au calcul d'indices représentatifs de l'activité musculaire des muscles profonds de la sangle abdominale via un module de calcul d'indices **43.**

Ces indices, ainsi que leurs évolutions temporelles sont ensuite visualisables sur un moniteur **50.** Ce moniteur **50** permet aussi de visualiser en temps réel les signaux acquis pendant l'étape d'acquisition I, ou de les visualiser une fois enregistrés dans la mémoire après l'étape II d'enregistrement, ou encore de visualiser les signaux $s_i(t)$ générés pendant l'étape III de traitement des signaux.

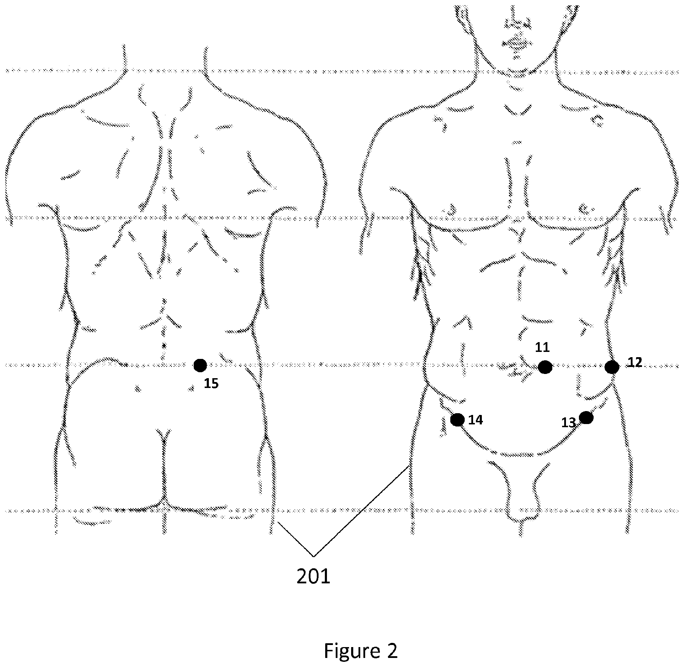

En référence à la figure 2, une implémentation particulière et non limitative de l'invention peut consister à positionner 5 électrodes de mesure EMG sur la surface de la peau d'un sujet **201** selon les sites anatomiques suivants :

- Une électrode **11** à 3 cm en latéral du nombril
- Une électrode **12** à 15 cm en latéral du nombril
- Une électrode **13** positionnée sous l'électrode **12,** juste au-dessus du ligament inguinal
- Une électrode **14** placée symétriquement à l'électrode **13**
- Une électrode **15** en face dorsale, sur une ligne reliant l'épine iliaque antéro-supérieure et l'espace intervertébral L1-L2, au niveau de l'apophyse épineuse L5.

La mise en œuvre décrite de l'invention sera identique pour :

- une électrode **11** placée entre 1 cm et 5 cm en latéral du nombril, vers la gauche ou bien vers la droite du sujet **201.**
- une électrode **12** placée entre 10 cm et 25 cm en latéral du nombril, vers la gauche ou vers la droite du sujet **201**
- une électrode **13** positionnée sous l'électrode **12,** à une distance comprise entre 4 cm et 15 cm, sans dépasser le ligament inguinal du sujet **201**

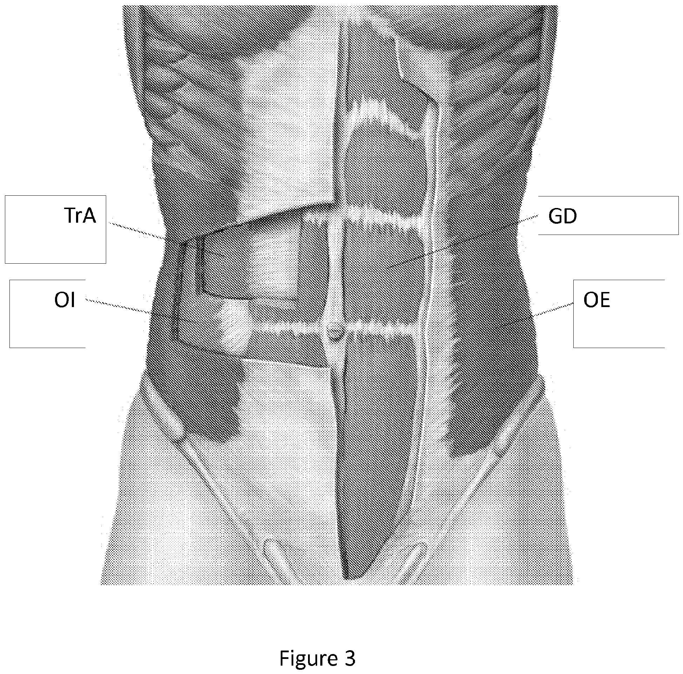

La figure 3 représente schématiquement de différents muscles de la sangle abdominale, composée 4 paires de muscles, qui sont organisés symétriquement de part et d'autre de l'abdomen sur trois couches distinctes. On retrouve donc parmi ces muscles abdominaux les paires suivantes :

- Noté **GD** sur la figure : Le Grand droit (le Grand Droit droit et le Grand Droit gauche). Constituant la paroi antérieure de l'abdomen, le muscle Grand Droit descend du thorax au pubis sous la forme d'un long muscle plat.
- Noté **OE** sur la figure : L'oblique externe, attaché sur les 7 dernières côtes, qui s'étend du centre de l'abdomen jusqu'au grand dorsal, occupant ainsi une bonne partie des côtés de l'abdomen.
- Noté **OI** sur la figure : L'oblique interne, qui s'insère sur toute la partie antérieure de la crête iliaque (comme le transverse, mais plus en surface)
- Noté **TrA** sur la figure : Le transverse, muscle large, le plus profond des muscles abdominaux. Situé dans la partie latérale de l'abdomen, le muscle transverse est horizontal, et passe ainsi derrière le muscle Grand Droit.

Grâce à ces éléments, le dispositif permet ainsi de mesurer en temps réel les signaux électriques issus de chaque électrode de mesure EMG de surface **11, 12, 13, 14 et 15,** chaque électrode mesurant ainsi une combinaison de toutes les contributions issues des activités musculaires des différents muscles étant à proximité de la dite électrode.

L'objectif de la méthode est de calculer des indices représentatifs de l'activité musculaire des muscles profonds de la sangle abdominale.

Dans l'exemple détaillé ici, le dispositif va ainsi calculer 3 indices :

$i_1$ : indice de contraction de l'oblique interne **OI**, égal à 1 si le muscle oblique interne est contracté, 0 sinon

$i_2$ : indice de contraction du transverse abdominal **TrA** égal à 1 si le muscle transverse abdominal est contracté, 0 sinon

$i_3$ : indice de la force de contraction du transverse abdominal **TrA,** proportionnel à la force de contraction du muscle transverse abdominal, et exprimé en pourcentage de la MCV pour ce muscle

(Maximum Contraction Value)

Ces indices et leurs évolutions temporelles seront calculés sur la même période d'échantillonnage que l'acquisition.

Pendant l'étape III de traitement du signal, la première étape, représentative de l'invention mais non limitative, consiste à appliquer une combinaison de différents algorithmes de traitement afin de nettoyer les signaux enregistrés. Chaque électrode conduit à un signal enregistré noté EMG($1_i$) pour i allant de 1 à 5 selon l'exemple non limitatif décrit.

Chaque signal EMG($1_i$) a donc été traité de la façon suivante :

- Redressement : il s'agit, après avoir évalué la ligne de base, puis corrigé le signal EMG($1_i$) de cet offset, de ne conserver que la valeur absolue du signal pour récupérer un signal strictement positif.
- Normalisation par rapport à des valeurs maximales mesurées sur des enregistrements obtenus lorsque le sujet effectue des exercices spécifiques qui permettent de conduire à des contractions quasi maximales des différents muscles de la sangle abdominale du sujet, dans la mesure de la capacité du sujet. Pour obtenir ces valeurs maximales, la méthode consiste à utiliser le dispositif pour enregistrer les signaux issus des électrodes pendant que le sujet effectue une manœuvre de « Draw-In » ou une manœuvre de « Crunch bloqué » par exemple.
- Lissage sur une fenêtre temporelle de 1ms : il s'agit de calculer la moyenne temporelle sur une fenêtre glissante de 1ms. La méthode peut être mise en œuvre avec une taille de fenêtre glissante comprise entre 0,5ms et 100ms.

Ces étapes conduisent donc à des signaux EMG(1i) modifiés, notés EMG'(1i) par la suite.

Dans l'exemple décrit, représentatif mais non limitatif de l'invention, il est supposé que les signaux des électrodes de surface sont une combinaison des contributions des 4 muscles de la sangle abdominale décrit sur la figure 3, à savoir le grand droit (**GD**), l'oblique externe (**OE**), l'oblique interne (**OI**) et le transverse abdominal (**TrA**). L'activité électrique de chacun de ces muscles peut être représentée par les expressions $A_{GD}$, $A_{OE}$, $A_{OI}$ et $A_{TrA}$, chaque électrode recevant donc une somme pondérée de ces activités à chaque instant (équation donnée pour l'électrode **11**):

$$EMG(11) = \alpha 11. A_{GD} + \beta 11. A_{OE} + \gamma 11. A_{OI} + \delta 11. A_{TrA}$$

Il s'agit donc ici d'un système de 5 équations à 20 paramètres et 4 inconnues, basé sur 5 enregistrements de 4 activités musculaires qui seront considérées comme indépendants statistiquement. La méthode mathématique consiste donc à déterminer les valeurs des 20 coefficients pondérateurs afin de pouvoir, en inversant le système par la suite, isoler soit l'activité électrique $A_{OI}$ permettant de calculer l'indice $i_1$, soit l'activité électrique $A_{TrA}$, permettant de calculer les indices $i_2$ et $i_3$.

Ceci a été effectué par une analyse de séparation de sources, implémentée dans le logiciel de calcul matriciel *Scilab* (marque déposée).

L'indice $i_1$ est ensuite calculé comme étant égal à 1 si l'amplitude du signal $A_{OI}$ est supérieure à un seuil fixé entre 5% et 15% de sa valeur maximale, 0 sinon.

L'indice $i_3$ est ensuite calculé comme étant égal à 1 si l'amplitude du signal $A_{TrA}$ est supérieure à un seuil fixé entre 5% et 15% de sa valeur maximale, 0 sinon.

L'indice $i_2$ est ensuite calculé comme étant un pourcentage de la valeur du signal $A_{TrA}$.

Le dispositif permet ainsi de visualiser en temps réel l'évolution de la valeur des indices lorsque que le sujet effectue des mouvements, ou des exercices de renforcement musculaire ou encore des exercices de rééducation.

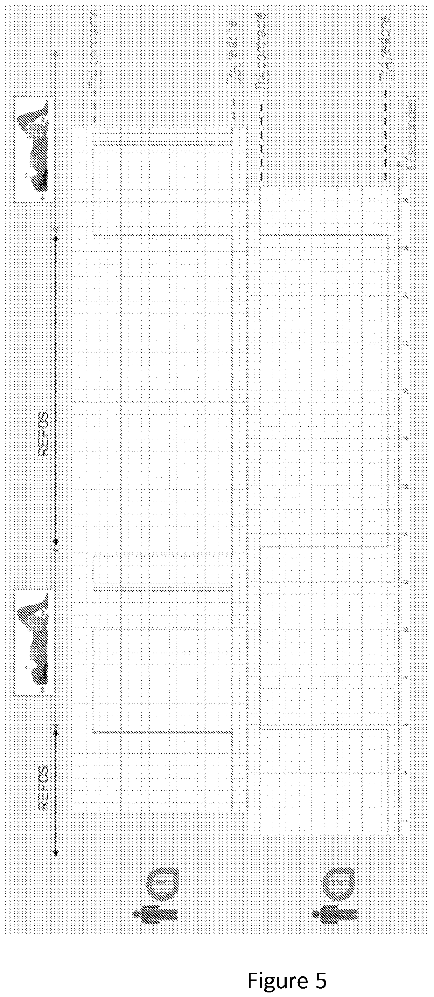

La figure 5 illustre ainsi la représentation de l'indice de contraction du muscle transverse abdominal $i_2$ pendant que le sujet effectue des manœuvres de Draw-In répétées, entre coupées de périodes de 10 s de repos.

Le dispositif permet aussi d'afficher en temps réel l'activité du muscle transverse abdominal suivant plusieurs modes : « on/off » (en représentant l'indice $i_2$), ou bien une visualisation de l'intensité de contraction en fonction du temps (indice $i_3$), pouvant se superposer à une consigne « idéale » qui doit être suivie par le sujet contrôlant ainsi de façon consciente la contraction de ses muscles.

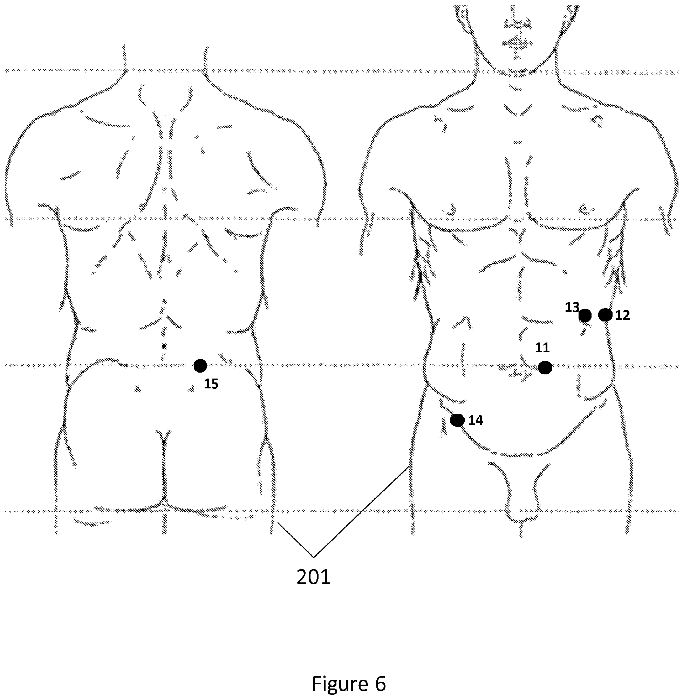

En référence à la figure 6, une implémentation particulière et non limitative de l'invention peut consister à positionner 5 électrodes de mesure EMG sur la surface de la peau d'un sujet **201** selon les sites anatomiques suivants :

- Une électrode **11** à 3 cm en latéral du nombril
- Une électrode **12** à 15 cm en latéral du nombril, puis en remontant jusqu'à la 8ème côte
- Une électrode **13** positionnée 2cm à gauche de l'électrode **12**, vers le centre du sujet
- Une électrode **14** positionnée à 15cm à droite du nombril su sujet, puis en redescendant jusqu'au ligament inguinal
- Une électrode **15** en face dorsale, sur une ligne reliant l'épine iliaque antéro-supérieure et l'espace intervertébral L1-L2, au niveau de l'apophyse épineuse L5.

La mise en œuvre décrite de l'invention sera identique pour :

- une électrode **11** placée entre 1 cm et 5 cm en latéral du nombril, vers la gauche ou bien vers la droite du sujet **201.**
- une électrode **12** placée entre 10 cm et 25 cm en latéral du nombril, vers la gauche ou vers la droite du sujet **201**
- une électrode **13** positionnée entre 1cm et 5 cm en latéral de l'électrode **12,** vers le centre du sujet

Dans un autre mode de réalisation, concernant un dispositif comprenant 5 électrodes positionnées à nouveau comme le montre la figure 2 ou encore comme le montre la figure 6, les signaux enregistrés par les électrodes de mesure EMG de surface **13** et **14** pourront être analysés également par une mesure de la fonction de corrélation temporelle afin de conditionner ensuite le calcul des indices $I_1$ et $I_2$.

L'indice $i_1$ sera égal à 1 si l'amplitude du signal $A_{OI}$ est supérieure à un seuil fixé entre 5% et 15% de sa valeur maximale ET si les deux signaux *EMG'*(13) et *EMG'*(14) sont faiblement corrélés, 0 sinon. L'indice $i_3$ est ensuite calculé comme étant égal à 1 si l'amplitude du signal $A_{TrA}$ est supérieure à un seuil fixé entre 5% et 15% de sa valeur maximale ET si les deux signaux *EMG'*(13) et *EMG'*(14) sont fortement corrélés, 0 sinon.

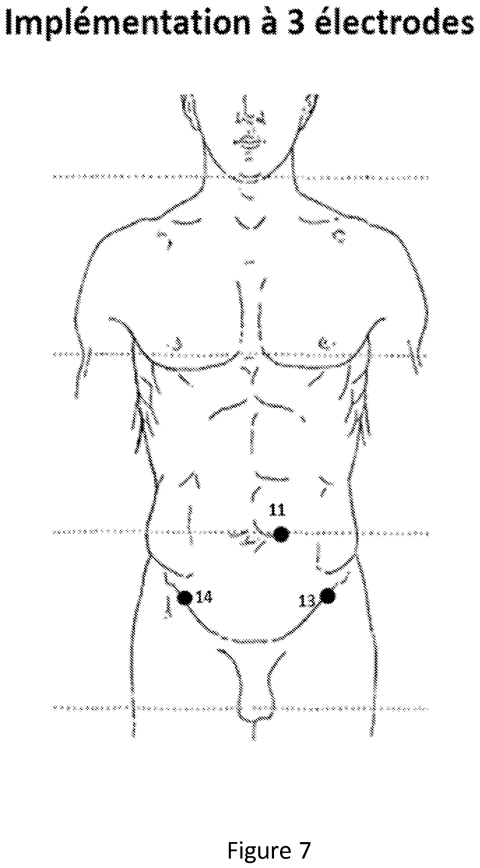

Dans un autre mode de réalisation non limitatif, concernant un dispositif comprenant 5 électrodes positionnées à nouveau comme le montre la figure 2, ou la figure 6, ou comprenant 3 électrodes comme décrit à la figure 7, les signaux EMG'(1i) sont analysés par l'application d'une analyse fréquentielle permettant de conditionner le calcul des indices $i_1$ et $i_3$. Les transformées de fourrier des signaux EMG'(1i) sont calculées pour représenter les spectres fréquentiels. On identifie ensuite une bande de fréquence caractéristique du muscle profond que l'on cherche à caractériser, ou un rapport de bandes de fréquence caractéristique pour ainsi conditionner les calculs des indices $I_1$ et $I_2$.

Par exemple, si $X_i(f)$ sont les transformées de Fourier respectives des signaux EMG'(1i), et $DSP_i$ les densités spectrales de puissance de ces transformées de fourrier, calculées sur un temps d'intégration adapté.

On opère alors le calcul suivant sur chaque voie de mesure :

$$Ri = \frac{\int_{f1}^{f2} DSPi}{\int_{0}^{fmax} DSPi}$$

Ou $f_1$ et $f_2$ sont deux fréquences de coupures, $f_1$ est comprise entre 0 et 100Hz, et $f_2$ comprise entre 50 et 200Hz. Les valeurs choisies pour ces deux fréquences de coupures varient d'une électrode à une autre.

Par exemple, pour les électrodes 11, 14 et 13, le mode de réalisation peut être mise en œuvre en choisissant $f_1$ = 65Hz et $f_2$ = 95Hz.

L'indice $i_1$ est ensuite calculé comme étant égal à 1 si les conditions suivantes sont communément réunies :

- $R_{14}$ et $R_{13}$ sont simultanément supérieures à un seuil fixé entre 0.55 et 0.8.
- $R_{11}$ est inférieure à un seuil compris entre 0.1 et 0.35.

Sinon, $i_1$ vaut 0.

Une implémentation particulière et non limitative peut consister à positionner 3 électrodes de mesure EMG sur la surface de la peau d'un sujet selon les sites anatomiques suivants :

- Une électrode **11** à 3 cm en latéral du nombril
- Une électrode **13** positionnée 2 cm en latéral de l'épine iliaque antéro-supérieure vers l'ombilic, puis 2 cm en longitudinal vers les pieds.
- Une électrode **14** placée symétriquement à l'électrode **13.**

Les modes de réalisation décrits ci-dessus sont particuliers et non limitatifs.

**Revendications**

1. Une méthode de mesure de l'activité musculaire individuelle simultanée de l'oblique interne et du transverse abdominal, et optionnellement d'un ou plusieurs muscles profonds additionnels de la sangle abdominale choisi(s) parmi le multifidus, les grands droits et l'oblique Externe, d'un sujet effectuant des exercices de renforcement musculaire à des fins esthétiques ou sportives à partir de capteurs électromyographiques surfaciques non invasifs (10) positionnés en un arrangement non circulaire le long du muscle ciblé comprenant, pour chaque muscle profond, les étapes (I) à (IV) suivantes :

a. Une étape (I) d'acquisition des signaux électriques issus d'au moins deux capteurs (10), la méthode étant **caractérisée par le fait que** le nombre de capteurs est compris entre 2 et 5,
b. Une étape (II) d'enregistrement des signaux électriques acquis pendant l'étape (I) dans une mémoire (41),
c. Une étape (III) de traitement des signaux enregistrés à l'étape (II) basée sur l'application sur les signaux enregistrés à l'étape (II) d'une combinaison d'algorithmes de traitement de signal dans un module de traitement de signal (42) comprenant un redressement, une normalisation, un lissage, un seuillage, et une analyse de séparation de sources, et délivrant ainsi une pluralité de signaux traités ($s_1(t)$, $s_2(t)$, $s_3(t)$....$s_n(t)$),
d. Une étape (IV) de calcul d'un indice à partir des signaux obtenus à l'étape c., représentatif de l'activité musculaire individuelle de chacun des au moins un muscle profond de la sangle abdominale du sujet, au moyen de l'exécution des algorithmes au sein d'environnements de calcul numérique ou au sein d'un processeur, et son évolution temporelle dans un module de calcul d'indice (43).

2. Méthode selon la revendication 1, comprenant une étape de visualisation des signaux acquis, enregistrés, traités, ou des indices calculés.

3. Méthode selon l'une des revendications 1 ou 2, dans laquelle les enregistrements de l'étape (I) sont effectués à une fréquence d'échantillonnage comprise en 500Hz et 3000Hz.

4. Méthode selon l'une des revendications 1 à 3, dans laquelle les capteurs (10) sont des électrodes EMG bipolaires.

5. Méthode selon l'une des revendications 1 à 4, dans laquelle la communication des signaux acquis pendant l'étape (I) vers la mémoire (41) est mise en œuvre par un procédé de communication de données sans fil.

**Patentansprüche**

1. Verfahren zum Messen der gleichzeitigen individuellen Muskelaktivität des inneren schrägen und des quer verlaufenden Bauchmuskels und gegebenenfalls eines oder mehrerer zusätzlicher tiefer Muskeln des Bauchgürtels, die aus dem Multifidus, dem Rectus abdominis und dem äußeren schrägen Muskel ausgewählt werden, bei einer Person, die Muskelstärkungsübungen zu ästhetischen oder sportlichen Zwecken durchführt, unter Verwendung von nicht-invasiven elektromyographischen Oberflächensensoren (10), die in einer nicht-kreisförmigen Anordnung entlang des anvisierten Muskels angeordnet sind, umfassend für jeden tiefen Muskel die folgenden Schritte (I) bis (IV)

a. Schritt (I) der Erfassung von elektrischen Signalen von mindestens zwei Sensoren (10), wobei das Verfahren **dadurch gekennzeichnet ist, dass** die Anzahl der Sensoren zwischen 2 und 5 liegt,
b. Ein Schritt (II) zur Aufzeichnung der in Schritt (I) erfassten elektrischen Signale in einem Speicher (41),
c. Schritt (III) des Verarbeitens der in Schritt (II) aufgezeichneten Signale auf der Grundlage der Anwendung einer Kombination von Signalverarbeitungsalgorithmen in einem Signalverarbeitungsmodul (42) auf die in Schritt (II) aufgezeichneten Signale, die eine Entzerrung, eine Normalisierung, eine Glättung, einen Schwellenwert und eine Quellentrennungsanalyse umfassen, und somit eine Vielzahl von verarbeiteten Signalen ($s_1(t)$, $s_2(t)$, $s_3(t)$....$s_n(t)$) liefern,
d. Schritt (IV) der Berechnung eines Indexes aus den in Schritt c. erhaltenen Signalen, der repräsentativ für die individuelle Muskelaktivität jedes einzelnen tiefen Muskels des Bauchgürtels des Probanden ist, mittels der Ausführung von Algorithmen innerhalb von digitalen Rechenumgebungen oder innerhalb eines Prozessors, und seiner zeitlichen Entwicklung in einem Indexberechnungsmodul (43)

2. Verfahren nach Anspruch 1, umfassend einen Schritt der Visualisierung der erfassten, aufgezeichneten und verarbeiteten Signale oder der berechneten Indizes.

3. Verfahren nach einem der Ansprüche 1 oder 2, bei dem die Aufnahmen in Schritt (I) mit einer Abtastfrequenz zwischen 500 Hz und 3000 Hz erfolgen.

4. Verfahren nach einem der Ansprüche 1 bis 3, bei dem die Sensoren (10) bipolare EMG-Elektroden sind.

5. Verfahren nach einem der Ansprüche 1 bis 4, bei dem die Übermittlung der im Schritt (I) erfassten Signale an den Speicher (41) durch ein drahtloses Datenübertragungsverfahren realisiert wird.

**Claims**

1.  A method of measuring the simultaneous individual muscle activity of the internal oblique and transverse abdominis, and optionally one or more additional deep muscles of the abdominal belt chosen from the multifidus, the rectus abdominis and the external oblique, of a subject performing muscle strengthening exercises for aesthetic or sporting purposes using non-invasive surface electromyographic sensors (10) positioned in a non-circular arrangement along the targeted muscle comprising, for each deep muscle, the following steps (I) to (IV)

    a. A step (I) of acquisition of electrical signals from at least two sensors (10), the method being **characterised by** the fact that the number of sensors is between 2 and 5,
    b. A step (II) for recording the electrical signals acquired during step (I) in a memory (41),
    c. A step (III) of processing the signals recorded in step (II) based on the application to the signals recorded in step (II) of a combination of signal processing algorithms in a signal processing module (42) comprising rectification, normalisation, smoothing, thresholding, and source separation analysis, and thus delivering a plurality of processed signals ($s_1(t)$, $s_2(t)$, $s_3(t)$....$s_n(t)$),
    d. A step (IV) of calculating an index from the signals obtained in step c., representative of the individual muscular activity of each of at least one deep muscle of the subject's abdominal belt, by means of the execution of algorithms within digital computing environments or within a processor, and its temporal evolution in an index calculation module (43).

2.  Method according to claim 1, comprising a step of visualisation of the acquired, recorded and processed signals or of the calculated indices.

3.  Method according to one of claims 1 or 2, in which the step (I) recordings are made at a sampling frequency between 500Hz and 3000Hz.

4.  Method according to any one of claims 1 to 3, in which the sensors (10) are bipolar EMG electrodes.

5.  Method according to any one of claims 1 to 4, in which the communication of the signals acquired during step (I) to memory (41) is implemented by a wireless data communication process.

Figure 1

Figure 2

Figure 3

Figure 4

Figure 5

Figure 6

# Implémentation à 3 électrodes

Figure 7

Figure 8

EP 3 709 878 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 9687168 B

**Littérature non-brevet citée dans la description**

- **MCGILL et al.** *J. Biomechanics*, 1996, vol. 29 (11), 1503-1507

- **JESINGER** ; **STONICK**. *6th IEEE DSP Workshop Proc.*, 1994, 57-60